# EUROPEAN PATENT SPECIFICATION

(11) **EP 2 913 014 B1**
(45) Date of publication and mention of the grant of the patent: **18.07.2018**
(21) Application number: 15156213.9
(22) Date of filing: 24.02.2015
(51) Int. Cl.: A61B 17/34, A61M 25/00

(54) **Overtube**
Überrohr
Surtube

(30) Priority: 27.02.2014 TW 103106687
(43) Date of publication of application: 02.09.2015
(73) Proprietor: ShineIN Biotechnology Co., Ltd., New Taipei City 242 (TW)
(72) Inventor: Dai, Lien-Guo, 406 Taichung (TW); Dai, Shiuan-De, 406 Taichung (TW)
(74) Representative: Lang, Christian

(56) References cited:
- CA-A- 628 292
- US-A- 4 498 902
- US-A- 4 765 334
- US-A- 4 846 791
- US-A- 5 279 553
- US-A- 5 807 317
- US-B1- 6 283 948
- US-B1- 6 589 212

## Description

### BACKGROUND OF THE INVENTION

### 1. FIELD OF THE INVENTION

This invention relates to a novel overtube, a kind of a sleeve, for use with an irrigation tip of a catheter in a suction-irrigation system for removing unwanted materials from a body cavity and delivering fluids thereto.

### 2. DESCRIPTION OF RELATED ART

Overtubes are common tools that may be used with surgical equipment to help guide the equipment to its intended position in a body. Accordingly, the overtubes are in general equipped with blades for making incisions on the surfaces of a body. Take the laparoscopic operation as an example, an overtube is used to make an incision on the abdomen of a subject, and the surgical tools such as drainage tube or endoscope may subsequently be introduced into the body of the subject.

Overtube may also be used with a drainage catheter, in which case, the drainage catheter is first inserted into the overtube, then an incision of the body is made by puncturing the body surface with the overtube having the drainage catheter inserted therein. After the incision is made, the overtube is then separated from the drainage catheter, in this way, the overtube help guide the draining catheter to its intended position in the body.

There are two types of overtubes available on the market, one is retractable overtube, and the other is dis-assemble or removal overtube. The retractable type of overtube is commonly used with an endoscopic surgical tool, and is doubled-layers in structure. It comprises an outer layer, and a pin for making a puncture. After the puncture is made by the pin, the pin is retracted, whereas the outer layer of the overtube will then serve as a conduit for delivering other surgical tools (e.g., the endoscope or the drainage catheter) into the body.

As to the dis-assemble or removal type of the overtube, it is generally designed to possess a built in mechanism that allows the overtube to be split in two halves when necessary, particularly during a surgery, so that the overtube may be completely removed and separated from the surgical tool. However, the choices of material suitable for making such type of overtubes are limited. The commercially available dis-assemble type of overtube is made by flexible plastic material that allows the overtube to be split in two halves easily, however, the flexible material also renders the creation of an incision on the body surface by the overtube difficult, if not impossible. Further, extra forces are required to split the overtube into two halves, which oftentimes cause unnecessary torn on the incision, or rendering it difficult for the attending physician to keep the irrigation tip in place.

CA 628 292 A discloses an overtube comprising a tubular body, in which a portion of the tubular body is cut away and the front end of the tubular body is tapered in a direction that is away from the cut away portion of the tubular body.

In view of the above, there exists in the related art an improved overtube that is free of the afore-mentioned problems commonly associated with the current commercially available overtube.

### SUMMARY

One object of this invention is to provide an improved overtube for use with an irrigation tip of a catheter during a wound irrigation process. The invention is described in claim 1. Preferred embodiments of the invention are described in the dependent claims. Also disclosed is a method for guiding an irrigation tip of a catheter in a wound irrigation process to its intended position for sterilely removing unwanted materials from a body cavity and delivering fluids thereto.

In one aspect, the present disclosure is directed to an overtube for use with an irrigation tip of an irrigation catheter during wound irrigation. The overtube comprising a tubular body, in which a portion of the tubular body has been cut away; and the frontend of the tubular body is tapered in a direction that is away from the cut away portion of the tubular body.

According to the present invention, the tapered frontend is at an angle about 130-175 degrees in relative to the tubular body.

According to optional embodiments of the present disclosure, the overtube further comprises a partition wall disposed within the overtube.

According to some embodiments of the present disclosure, the partition wall is slantingly disposed within the overtube.

According to further embodiments of the present disclosure, the tubular body having a cut away portion has a cross section that is about 1/3 to 2/3 of a circle. In some preferred embodiments, the cross section is about 1/2 of a circle.

According to optional embodiments of the present disclosure, the overtube further comprises a through-bore connecting the tip of the tapered frontend and the partition wall.

According to other optional embodiments of the present disclosure, the overtube further comprises a through-bore connecting the tip of the tapered frontend and the slant partition wall.

According to still other optional embodiments of the present disclosure, the overtube further comprises two protruding parts respectively disposed on the opposite sides of the outer surface of the rear end of the tubular body.

According to preferred embodiments of the present disclosure, the overtube is a one-piece article made from a metallic or a plastic material. The metallic material may be selected from the group consisting of, stainless steel, titanium, and an alloy comprising titanium, stainless steel or both. The plastic material may be selected from the group consisting of, polypropylene, polyvinylchloride, and high density polyethylene. In one example, the overtube is a one-piece article made from an alloy comprising titanium. In another example, the overtube is a one-piece article made from stainless steel. In still another example, the overtube is a one-piece article made from high density polyethylene.

In another aspect, the present disclosure is directed to an assembly for wound irrigation. The assembly comprises the overtube of the present disclosure, and an irrigation tip of an irrigation catheter.

### BRIEF DESCRIPTION OF THE DRAWINGS

The present description will be better understood from the following detailed description read in light of the accompanying drawings, wherein:
FIGs 1A to 1C are schematic drawings respectively illustrating an overtube for use with an irrigation tip during wound irrigation according to one embodiment of this invention;
FIGs 2A to 2C are schematic drawings respectively illustrating an overtube for use with an irrigation tip during wound irrigation according to another embodiment of this invention;
FIGs 3A to 3C are schematic drawings respectively illustrating a tapered frontend of an overtube according to a further embodiment of this invention;
FIG 4 illustrates another embodiment of the overtube of the present invention;
FIG 5 is a schematic drawing illustrating an assembly for wound irrigation in accordance with one embodiment of the present invention; and
FIGs 6 to 8 are schematic drawings illustrating the process of guiding an irrigation tip to its intended position in a tissue with the aid of the overtube of the present invention.

Like reference numerals are used to designate like parts in the accompanying drawings.

### DETAILED DESCRIPTION OF PREFERRED EMBODIMENTS

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

The singular forms "a", "and", and "the" are used herein to include plural referents unless the context clearly dictates otherwise.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The present disclosure aims at providing an overtube suitable for use with an irrigation tip (e.g., bi-lumen tip) of a catheter during wound irrigation to remove unwanted materials from a body cavity and delivering fluids thereto. The overtube may help guide the irrigation tip to the body cavity and may be easily removed once the irrigation tip has reached its intended position in the body cavity and thereby improves the efficiency of wound irrigation.

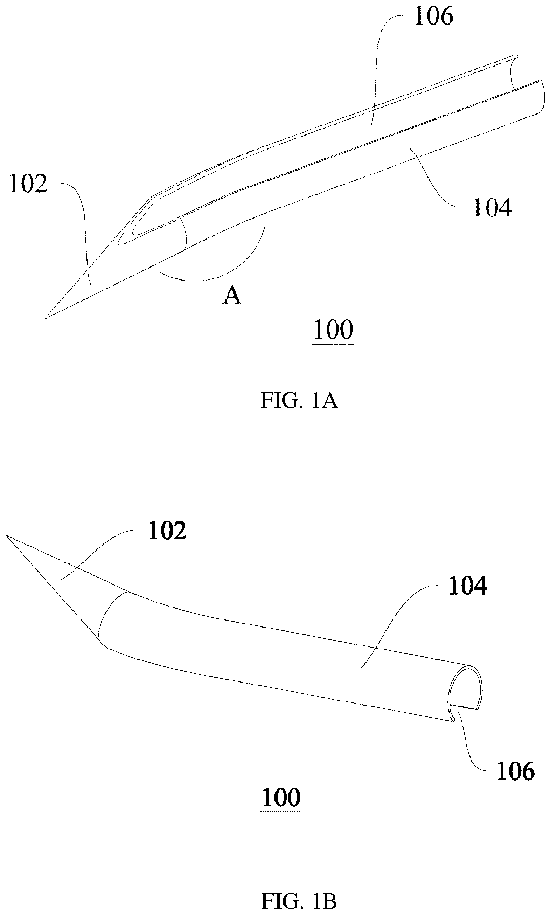

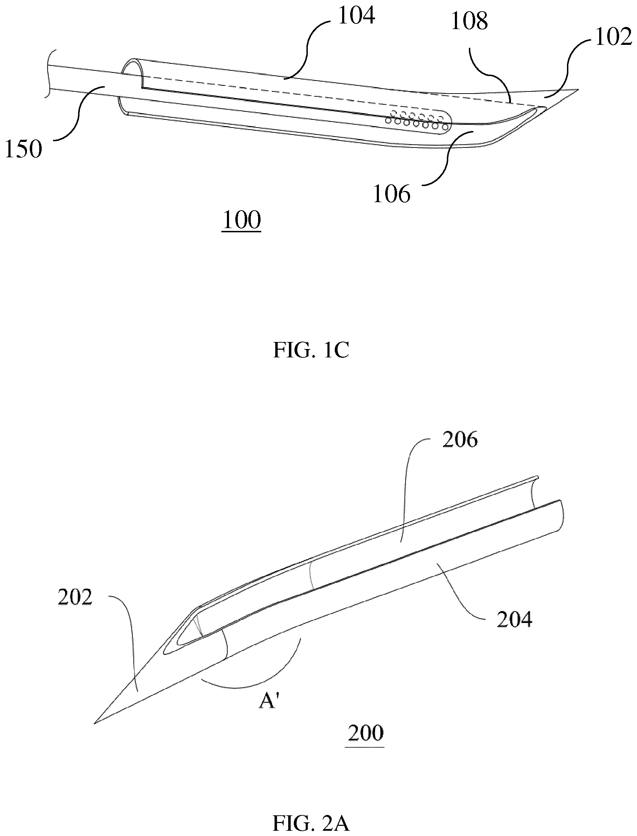

References are made to **FIGs 1A to 1C****,** in which **FIGs 1A** and **1B** are schematic drawings of the overtube 100 in according to one embodiment of the present disclosure, and **FIG 1C** is a schematic drawing illustrating the assembly of the overtube 100 and an irrigation tip 150 for wound irrigation.

The overtube 100 of this embodiment comprises in its structure, a tubular body 104, in which a portion of the tubular body 104 is cut away (the cut away portion is indicated as 106) for receiving the irrigation tip 150 of an irrigation catheter therein during a wound irrigation process **(****FIG 1C**); and the frontend 102 of the tubular body 104 is tapered and is at an obtuse angle A in relative to the tubular body 104 **(****FIG 1A**), particularly, the frontend 102 is tapered in a direction that is away from the cut away portion 106 of the tubular body 104 **(****FIG 1B**). The tapered frontend 102 may be used to puncture or make an incision on an intended portion of a body, such as on the skin above a body cavity (e.g., abscess cavity), and to help guide the irrigation tip 150 of the irrigation catheter into the body cavity to remove unwanted materials from the body cavity and delivering fluids thereto.

In general, the size of the overtube 100 should be sufficient enough to receive the irrigation tip 150 therein form the cut away portion 106 of the tubular body 104, and to allow the overtube 100 to be separated from the irrigation tip 150 once the irrigation tip 150 is in its intended position. According to some embodiments, the overtube 100 has an inner diameter of about 5-20 mm, such as 5, 6, 7, 8, 9, 10, 11, 12, 13, 14, 15, 16, 17, 18, 19, and 20 mm; preferably about 5-10 mm, such as 5, 6, 7, 8, 9, and 10 mm. Further, the overtube 100 has a cross section that is about 1/3 - 2/3 of a circle. Preferably, the cross section of the overtube 100 is about one-half of a circle.

As depicted in **FIG 1A****,** the tapered frontend 102 is at an obtuse angle A in relative to the tubular body 104. According to the invention, the obtuse angle A is about 130-175 degrees, such as 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, and 175 degrees; preferably about 135-170 degrees, such as 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, and 170 degrees.

According to optional embodiments, the overtube 100 of the present disclosure may further include a partition wall 108 disposed within the overtube 100, particularly between the tapered frontend 102 and the cut away portion 106 of the tubular body 104 (**FIG 1C**).

Before starting wound irrigation, the irrigation tip 150 of an irrigation catheter is inserted into the tubular body 104 from the cut away portion 106, it is then pressed against the partition wall 108 so as to hold the irrigation tip 150 in place when the user tries to create an incision or puncture the intended portion of the body with the tapered frontend 102 of the overtube 100.

In optional embodiments, a guide wire may be used to help guide the irrigation tip 150 to its intended position, accordingly, in some optional embodiments, the overtube 100 further comprises a through-bore (not depicted), connecting the tip of the tapered frontend 102 and the partition wall 108, and allows the guide wire to pass through (not depicted).

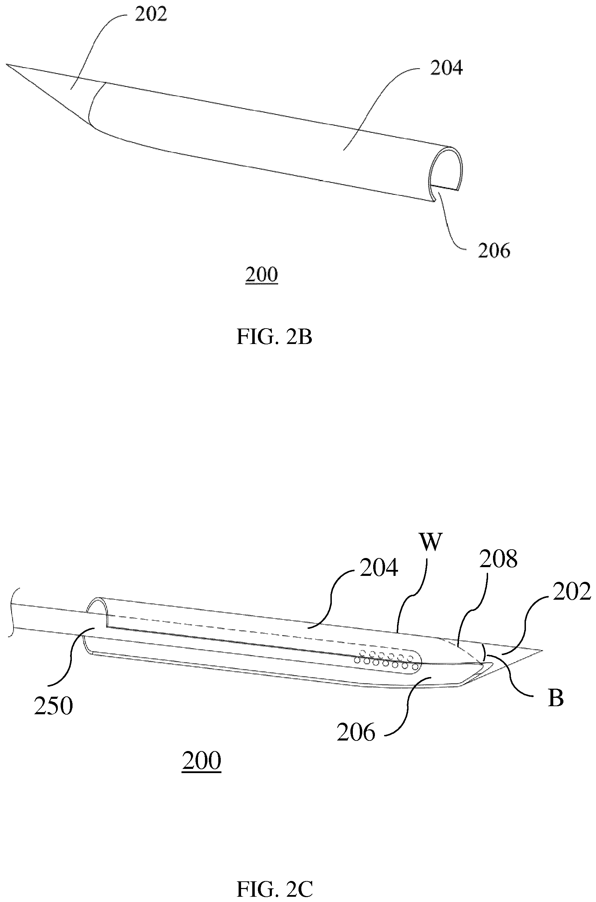

**FIGs 2A** and **2B** are schematic drawings depicting another embodiment of the overtube 200 of the present disclosure, and **FIG 2C** illustrates the assembly of the overtube 200 and an irrigation tip 250 for use in the wound irrigation process.

Similar to the overtube 100 described above, the overtube 200 has a tapered frontend 202 and a tubular body 204, in which a portion of the tubular body 204 has been cut away (the cut away portion is indicated as 206); and the tapered frontend 202 is at an obtuse angle A' in relative to the tubular body 204, particularly, the frontend is tapered in a direction that is away from the cut away portion 206 of the tubular body 204 **(****FIG 2A**). As depicted in **FIG 2A** **and** **2B****,** the tapered frontend 202 of the tubular body 204 is at 180 degrees in relative to the tubular body 204.

According to some optional embodiments, the overtube 200 of the present disclosure may further include a partition wall 208 disposed within the overtube 200, particularly the partition wall 208 is slantingly disposed and is at an angle B in relative to one side (W) of the tubular body 204 **(****FIG 2C**). According to some embodiments, the angle B may be about 5-90 degrees, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and 90 degrees; preferably about 10-85 degrees, such as 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, and 85 degrees. The slant partition wall 208 is designed to allow easy separation of the overtube 200 and the irrigation tip 250 received therein once the irrigation tip 250 has been inserted into a proper position (e.g., within the abscess cavity), then while holding the irrigation tip 250 at its place, the user may gently push the overtube 200 against the slant partition wall 208 and thereby allows the irrigation tip 250 to slide out from the cut away portion 206 of the tubular body 200, and the overtube 200 is separated from the irrigation tip 250.

According to optional embodiments, a guide wire may be used to help guide the irrigation tip 250 to its intended position (e.g., the abscess cavity), accordingly, in some optional embodiments, the overtube 200 may further include a through-bore (not depicted) disposed between the tip of the tapered frontend 202 and the partition wall 208 that allows the guide wire to be inserted there through.

As described above, the tapered frontend of the overtube of the present disclosure may be used to puncture or create an incision on an intended portion of the body, accordingly, in some embodiments, the tapered frontend is designed to equip with blades for such purpose.

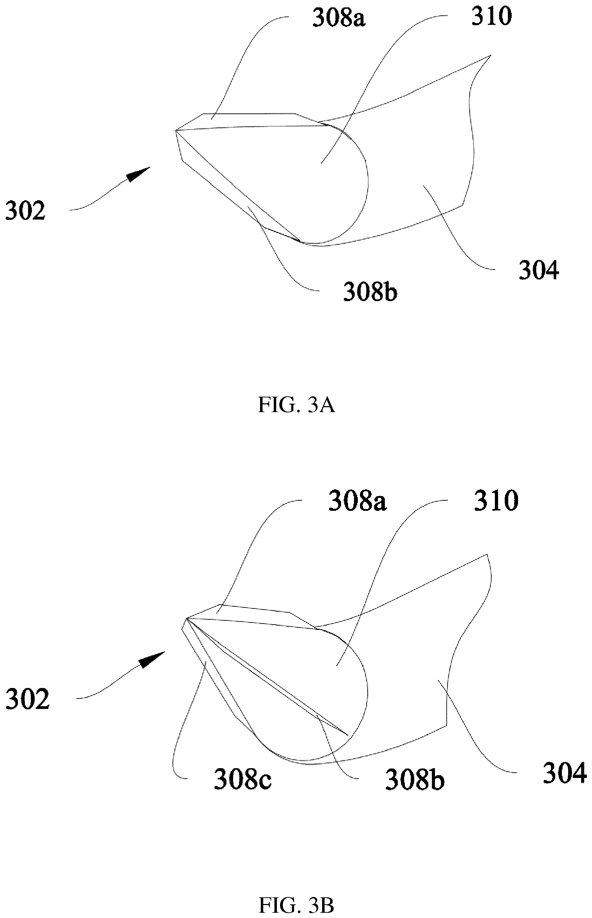

References are now made to **FIGs 3A to 3C****,** in which an overtube 310 comprising a tubular body 304 and a tapered frontend 302 having at least one blade for making an incision on the intended portion of the body are depicted.

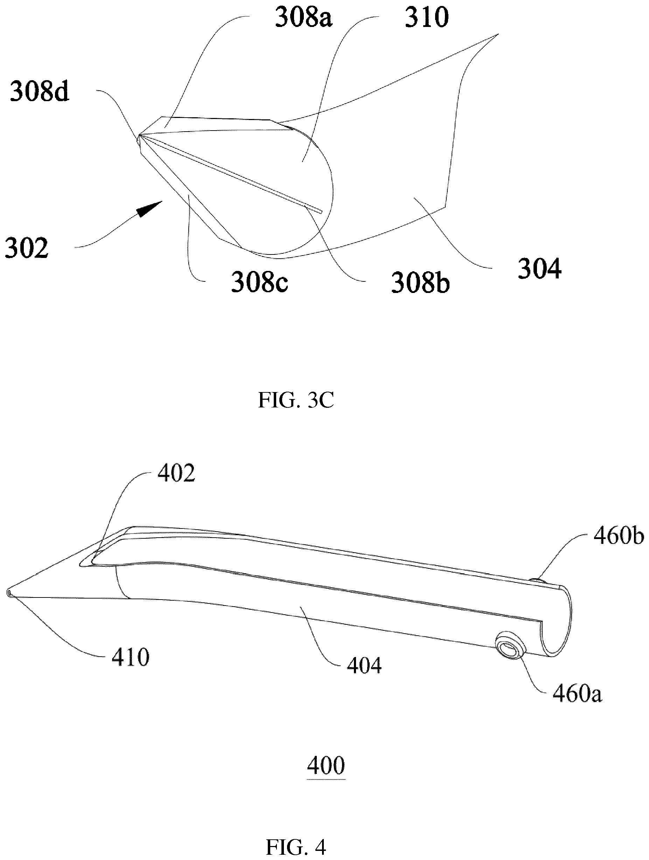

**FIG 3A** is a schematic drawing of the overtube 310, which comprises a tubular body 304 and a tapered frontend 302 having 2 blades (308a, 308b), in which the two blades are respectively disposed along one sides of the frontend 302. Optionally, the tapered frontend 302 may equip with more blades, such as 3 blades (308a, 308b, and 308c) as depicted in **FIG 3B**; or 4 blades (308a, 308b, 308c, and 308d) arranged in a cross-wise manner as depicted in **FIG 3C**.

**FIG 4** illustrates another optional embodiment of the overtube 400 of the present disclosure. In this optional embodiment, the overtube 400 further comprises in its structure, a through-bore 410 disposed at the tip of the tapered frontend 402 and two protruding parts (460a, 460b). The through-bore 410 allows a guide wire to travel there through and thereby helps the user to guide the irrigation tip to its intended position. Preferably, the through-bore 410 has an inner diameter of about 1 to 5 mm, such as 1, 2, 3, 4, and 5 mm. The two protruding parts (460a, 460b) are disposed on the opposite sides of the outer surface of the rear end of the tubular body 404, and are designed to allow the user a firm grip of the overtube 400. Instead of gripping on the smooth and slippery surface of the overtube 400, the user may hold the overtube 400 respectively at the two protruding parts (460a, 460b) by his/her two fingers.

In general, the overtube of the present disclosure is a one-piece article made from a metallic or plastic material. In some embodiments, the overtube is made from a metallic material selected from the group consisting of, stainless steel, titanium, and an alloy comprising titanium, stainless steel or both. In other embodiments, the overtube is made from a plastic material selected from the group consisting of, polypropylene, polyvinylchloride, and high density polyethylene. In one example, the overtube is a one-piece article made from an alloy comprising titanium. In another example, the overtube is a one-piece article made from stainless steel. In still another example, the overtube is a one-piece article made from high density polyethylene. In the case when the overtube is made from a metallic material, it may be re-used after sterilization. In the case when the overtube is made from a plastic material, it may be disposed after each use.

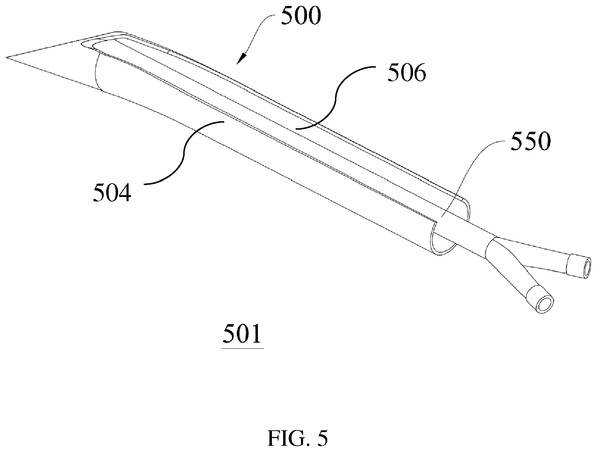

A further objective of the present disclosure aims at providing an irrigation assembly 501 for wound irrigation as depicted in **FIG 5**. The irrigation assembly 501 is composed of an overtube 500, and a bi-lumen tip 550 of an irrigation catheter. The bi-lumen tip 550 may be any bi-lumen tip suitable for irrigation purpose, such as the bi-lumen tip disclosed in WO2012151751. As illustrated in **FIG 5****,** before the irrigation process starts, the bi-lumen tip 550 is assembled with the overtube 500 by inserting the bi-lumen tip 550 into the overtube 500 from the cut away portion 506 of the tubular body 504. Once the bi-lumen tip 550 has been guided and reached its intended position (e.g., the abscess cavity), the assembly is disassembled by pushing the overtube 500 away from the bi-lumen tip 550 and thereby allows the bi-lumen tip 550 to slide out from the cut away portion 506 of the tubular body 504.

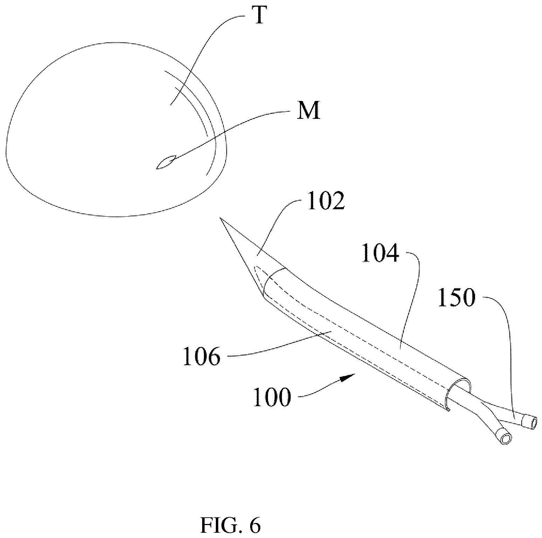

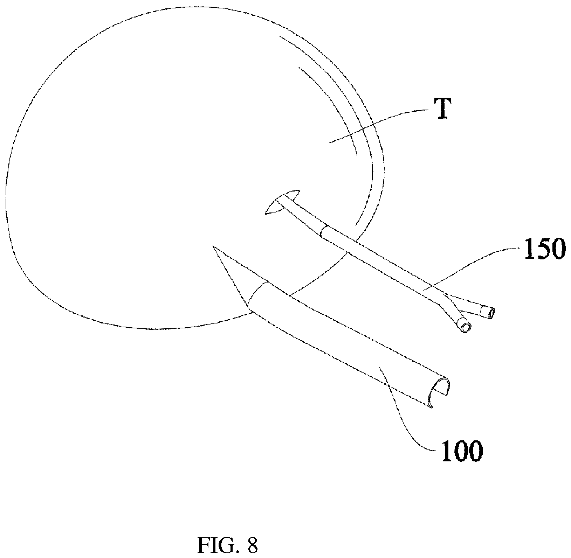

References are now made to **FIGs 6 to 8****,** which depict a wound irrigation process using the overtube 100 of **FIG 1**.

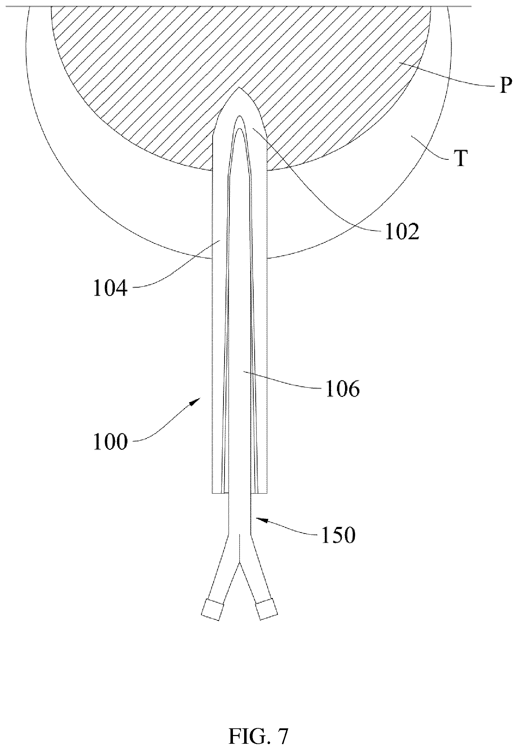

Before wound irrigation starts, the overtube 100 is first assembled with a bi-lumen tip 150 of an irrigation catheter by inserting the bi-lumen tip 150, which is coupled to an irrigation system (not depicted), into the overtube 100 from the cut away portion 106 of the tubular body 104. Then, while holding the bi-lumen tip 150 at its place, the intended body position for making an incision (indicated as M) was punctured or cut through using the tapered frontend 102 of the overtube 100; the bi-lumen tip 150 is then pushed into the cut tissue (as indicated by T) until it reaches its intended place (indicated as P) **(****FIG 7**). Once the bi-lumen tip 150 is in its place inside the tissue (e.g., abscess cavity), the overtube 100 is pushed away from the bi-lumen tip 550 and thereby allows the bi-lumen tip 150 to slide out from the cut away portion 106 of the tubular body 104. At this stage, the bi-lumen tip 150 would be at its place, and the wound irrigation process may then begin to remove unwanted body debris or fluids from the wound cavity by use of a suction-irrigation system commonly known by any skilled artisan in the related art.

The overtube of the present disclosure provides a unique and useful tool for use with irrigation tip of any conventional irrigation system and/or method for conditions where drainage of unwanted materials and delivery of medical fluids are required with a minimum of complications.

It will be understood that the above description of embodiments is given by way of example only and that various modifications may be made by those with ordinary skill in the art. For example, the bi-lumen tip having first and second lumens may be tapered toward one end and is bifurcated at the other end, such that the two lumens of the bi-lumen tip share a common lumen wall at the perforated end and remain separated at the bifurcated end. During the suction-irrigation process, the perforated end of the bi-lumen tip is inserted into a body cavity of a patient, whereas the bifurcated end, in which the two lumens remain separated, is coupled to the suction unit and the fluid delivery unit respectively through suitable catheters, and thereby forming an irrigation system for removing unwanted materials from the body cavity and delivering fluids thereto.

## Claims

1. An overtube (100) for use with an irrigation tip of an irrigation catheter (150) during wound irrigation comprising a tubular body (104), in which
a portion of the tubular body (104) is cut away; and the frontend (102) of the tubular body (104) is tapered in a direction that is away from the cut away portion of the tubular body (104), wherein the tapered frontend (102) is at an angle of about 130 to 175 degrees relative to the tubular body (104).

2. The overtube of claim 1, further comprising a partition wall (108) disposed within the overtube (100).

3. The overtube of claim 2, wherein the partition wall (108) is slantingly disposed within the overtube (100).

4. The overtube of claim 1, wherein the tubular body (100) having a cut away portion has a cross section that is about 1/3 to 2/3 of a circle.

5. The overtube of claim 4, wherein the cross section is about 1/2 of a circle.

6. The overtube of claim 2, further comprising a through-bore connecting the tip of the tapered frontend (102) and the partition wall (108).

7. The overtube of claim 3, further comprising a through-bore connecting the tip of the tapered frontend (102) and the slant partition wall (108).

8. The overtube of claim 6, further comprising two protruding parts (460a, 460b) respectively disposed on the opposite sides of the outer surface of the rear end of the tubular body (104).

9. The overtube of claim 7, further comprising two protruding parts (460a, 460b) respectively disposed on the opposite sides of the outer surface of the rear end of the tubular body (104).

10. The overtube of claim 1, wherein the overtube (100) is a one-piece article made from a metallic or a plastic material.

11. The overtube of claim 10, wherein the metallic material is any of stainless steel, titanium, or an alloy comprising stainless steel, titanium or both.

12. The overtube of claim 10, wherein the plastic material is any of polypropylene, polyvinylchloride, or high density polyethylene.

13. An assembly for wound irrigation, comprising:
the overtube (100) of claim 1; and
an irrigation tip of an irrigation catheter (150);
wherein, the cut away portion of the tubular body (104) allows the irrigation tip to be received therein and/or removed therefrom.

## Patentansprüche

1. Überrohr (100) zum Gebrauch mit einer Irrigationsspitze eines Irrigationskatheters (150) während der Wundspülung, umfassend einen rohrförmigen Körper (104), bei welchem ein Teil des rohrförmigen Körpers (104) weggeschnitten ist und bei welchem die Frontpartie (102) des rohrförmigen Körpers (104) in eine Richtung weg von dem weggeschnittenen Teil des rohrförmigen Körpers konisch verläuft, wobei die spitz zulaufende Frontpartie (102) in einem Winkel von 130 bis 175 Grad relativ zu dem rohrförmigen Körper (104) verläuft.

2. Überrohr nach Anspruch 1, weiterhin umfassend eine Trennwand (108), die innerhalb des Überrohres angeordnet ist.

3. Überrohr nach Anspruch 2, bei welchem die Trennwand (108) schräg innerhalb des Überrohres (108) angeordnet ist.

4. Überrohr nach Anspruch 1, bei welchem der rohrförmige Körper (104) mit dem weggeschnittenen Teil einen Querschnitt von etwa 1/3 bis 2/3 eines Kreises aufweist.

5. Überrohr nach Anspruch 4, bei welchem der Querschnitt ungefähr 1/2 eines Kreises ist.

6. Überrohr nach Anspruch 2, weiterhin umfassend eine Durchgangsbohrung, die die Spitze der spitzzulaufenden Frontpartie (102) und die Trennwand (108) verbindet.

7. Überrohr nach Anspruch 3, weiterhin umfassend eine Durchgangsbohrung, die die Spitze der spitzzulaufenden Frontpartie (102) und die schräge Trennwand (108) verbindet.

8. Überrohr nach Anspruch 6, weiterhin umfassend zwei vorstehende Teile (460a, 460b), die jeweils an den gegenüberliegenden Seiten der Außenseite des hinteren Endes des rohrförmigen Körpers (104) angeordnet sind.

9. Überrohr nach Anspruch 7, weiterhin umfassend zwei vorstehende Teile (460a, 460b), die jeweils an den gegenüberliegenden Seiten der Außenseite des hinteren Endes des rohrförmigen Körpers (104) angeordnet sind.

10. Überrohr nach Anspruch 1, bei welchem das Überrohr (100) ein einstückiger Gegenstand ist, der aus einem metallischen Material oder einem Plastikmaterial hergestellt ist.

11. Überrohr nach Anspruch 10, bei welchem das metallische Material irgendeines aus Edelstahl, Titan oder einer Legierung ist, die Edelstahl, Titan oder beides umfasst.

12. Überrohr nach Anspruch 10, bei welchem das Plastikmaterial irgendeines aus Polypropylen, Polyvinylchlorid, oder hochmolekularem Polypropylen (HDPE) ist.

13. Anordnung für die Wundspülung, umfassend:
das Überrohr (100) aus Anspruch 1 und
eine Irrigationsspitze von einem Irrigationskatheter (150),
bei welcher das weggeschnittene Teil des rohrförmigen Körpers (104) der Irrigationsspitze erlaubt, darin aufgenommen und/oder davon entfernt zu werden.

## Revendications

1. Surtube (100) pour utilisation avec un embout d'irrigation d'un cathéter d'irrigation (150) au cours de l'irrigation d'une plaie, comprenant un corps tubulaire (104), dans lequel:
une partie du corps tubulaire (104) est découpée; et l'extrémité frontale (102) du corps tubulaire (104) est amincie dans une direction qui s'écarte de la partie découpée du corps tubulaire (104), dans lequel:
l'extrémité frontale amincie (102) se situe sous un angle d'environ 130 à 175 degrés par rapport au corps tubulaire (104).

2. Surtube selon la revendication 1, comprenant en outre une paroi de séparation (108) disposée dans le surtube (100).

3. Surtube selon la revendication 2, dans lequel la paroi de séparation (108) est disposée en oblique dans le surtube (100).

4. Surtube selon la revendication 1, dans lequel le corps tubulaire (100) ayant une partie découpée a une section transversale qui est d'environ 1/3 à 2/3 d'un cercle.

5. Surtube selon la revendication 4, dans lequel la section transversale est d'environ ½ d'un cercle.

6. Surtube selon la revendication 2, comprenant en outre un trou traversant raccordant l'embout de l'extrémité frontale amincie (102) et la paroi de séparation (108).

7. Surtube selon la revendication 3, comprenant en outre un trou traversant raccordant l'embout de l'extrémité frontale amincie (102) et la paroi de séparation oblique (108).

8. Surtube selon la revendication 6, comprenant en outre deux parties saillantes (460a, 460b) respectivement disposées sur les côtés opposés de la surface externe de l'extrémité arrière du corps tubulaire (104).

9. Surtube selon la revendication 7, comprenant en outre deux parties saillantes (460a, 460b) respectivement disposées sur les côtés opposés de la surface externe de l'extrémité arrière du corps tubulaire (104).

10. Surtube selon la revendication 1, dans lequel le surtube (100) est un article d'un seul tenant constitué d'un matériau métallique ou d'une matière plastique.

11. Surtube selon la revendication 10, dans lequel le matériau métallique est l'un quelconque des suivants: de l'acier inoxydable, du titane ou un alliage d'acier inoxydable, de titane ou des deux.

12. Surtube selon la revendication 10, dans lequel la matière plastique est l'une quelconque des suivantes : polypropylène, poly(chlorure de vinyle) ou polyéthylène de haute densité.

13. Ensemble pour irrigation d'une plaie, comprenant:
le surtube (100) selon la revendication 1; et
un embout d'irrigation d'un cathéter d'irrigation (150);
dans lequel la partie découpée du corps tubulaire (104) permet d'y recevoir l'embout d'irrigation et/ou de l'en retirer.
